# EUROPEAN PATENT SPECIFICATION

(11) **EP 3 433 012 B1**
(45) Date of publication and mention of the grant of the patent: **05.08.2020**
(21) Application number: 17709424.0
(22) Date of filing: 07.03.2017
(51) Int. Cl.: B01J 27/04, C07C 5/32, C07C 11/04, C07C 11/06, C07C 11/08, C07C 11/09

(54) **LEAD SULFIDE AS ALKANE DEHYDROGENATION CATALYST**
BLEISULFID ALS ALKAN-DEHYDRIERUNGSKATALYSATOR
SULFURE DE PLOMB EN TANT QUE CATALYSEUR DE DÉSHYDROGÉNATION D'ALCANES

(30) Priority: 22.03.2016 DK 201600175
(43) Date of publication of application: 30.01.2019
(73) Proprietor: Haldor Topsøe A/S, 2800 Kgs. Lyngby (DK)
(72) Inventor: HØJLUND NIELSEN, Poul Erik, 3480 Fredensborg (DK); NIELSEN, Rasmus Munksgård, 2760 Måløv (DK); LEMUS-YEGRES, Lived J., 2300 Copenhagen S (DK)
(74) Representative: Haldor Topsøe A/S
(86) International application number: PCT/EP2017/055289
(87) International publication number: WO 2017/162431

(56) References cited:
- EP-A2- 0 568 303
- US-A- 2 315 107
- US-A- 2 768 931

## Description

The present invention relates to the use of lead sulfide as catalyst in processes for the dehydrogenation of alkanes to the corresponding alkenes.

Basically, the catalytic dehydrogenation of lower alkanes is a simple, but yet important reaction, which can be illustrated by the dehydrogenation of propane to propene in accordance with the reaction:

C₃H₈ <-> C₃H₆ + H₂

With the ever growing demand for light olefins, i.e. lower aliphatic open-chain hydrocarbons having a carbon-carbon double bond, catalytic dehydrogenation is growing in importance. Especially the dehydrogenation of propane and isobutane are important reactions used commercially for the production of propylene and isobutylene, respectively. Propylene is an important basic chemical building block for plastics and resins, and the worldwide demand for propylene has been growing steadily for decades. It is expected that the demand growth for propylene will soon be equal to or even higher than that for ethylene. One of the major applications of isobutylene is the use thereof as feedstock in the manufacture of methyl-tert-butyl ether (MTBE).

The process shown above is endothermic and requires about 125 kJ/mole in heat of reaction. Thus, in order to achieve a reasonable degree of conversion, the dehydrogenation process is taking place at a temperature around 600°C. The dehydrogenation of isobutane is similar to that of propane in every respect, apart from requiring a slightly lower temperature.

There are 3-4 commercial processes for alkane dehydrogenation in existence, using three different catalysts. The differences between these processes primarily deal with the supply of the heat of reaction. The processes and the catalysts are briefly described below.

### a) The Catofin (Houdry) process

This process is characterized by the heat of reaction being supplied by pre-heating of the catalyst. The Catofin process is carried out in 3-8 fixed bed adiabatic reactors, using a chromium oxide/alumina catalyst containing around 20 wt% chromium oxide. The catalyst may be supplemented with an inert material having a high heat capacity, or alternatively with a material which will selectively combust or react with the hydrogen formed, the so-called heat generating material (HGM). Promoters such as potassium may be added.

The Catofin process is a very well-established process and still the dominant industrial dehydrogenation process. Since the reaction heat is supplied by the catalyst, a sequential operation is used, during which the catalyst bed is used for dehydrogenation. Then the gas is purged away, and the catalyst is being regenerated/heated and the Cr(VI) oxide reduced with hydrogen. Finally the bed is purged with steam before another dehydrogenation.

### b) The Snamprogetti-Yarzintez process

This process is a fluid-bed version of the above process, using twin fluidized beds, one each on process and regeneration duty with catalyst cycling between them. Numerous plants are in operation, e.g. in the former Soviet Union and in Saudi Arabia.

The catalyst deactivation may be due to mechanical reasons, stress induced during heating-cooling cycles and solid state reactions, such as diffusion of chromium into the alumina lattice. This is, however, secondary to the desire to get rid of chromium, which is the real challenge in this process because the toxicity of chromium is a problem. More specifically, the presence of chromium in the catalyst makes it an environmental and health hazard to handle. This is particularly so because chromium(VI)oxide, CrO₃, and related compounds of chromium in oxidation state VI are easily formed by oxidation of the catalyst. Thus, every kind of handling of the catalyst during manufacture, transportation, loading and unloading is a potential hazard, and with the increasing demand for dehydrogenation processes it is desirable to find effective, less toxic dehydrogenation catalysts.

### c) The Oleflex process

The Oleflex process employs noble metal catalysts, especially a promoted Pt/Al₂O₃ catalyst in a reaction system of 3-4 moving bed reactors with the catalyst being continuously regenerated in a separate regeneration circuit. The heat of reaction is supplied by pre-heating the hydrocarbon stream. The noble metal catalyst is subject to slow deactivation. Thus, in the Oleflex process, the catalyst moves down in the radial flow bed. In the bottom, the catalyst is transported to a regeneration reactor, where the carbon on the catalyst is burned away and the platinum is dispersed again by means of a chlorine treatment. The regenerated catalyst is recycled back into the top of the dehydrogenation reactor. The cycle time is up to one week.

The noble metal is supported on an alumina carrier, and it is stabilized by means of tin and possibly other promoters. Platinum is a good catalyst choice from a technical point of view and it forms stable alloys with tin. The main problem with this kind of catalyst is the high price, which is currently counteracted by aiming to decrease the platinum loading.

### d) The STAR process

The STAR® process (STAR being an acronym for STeam Assisted Reforming) is a commercially established dehydrogenation technology, which has some attractive features.

Steam is being used as a diluent, and the process takes place in a tubular reactor like a steam reformer placed in a furnace. The reaction heat is supplied by firing with natural gas. The catalyst is Pt supported on a ZnAl₂O₄ spinel. Zn and Pt form some very stable alloys. Some carbon deposition takes place, and the catalyst must be regenerated every eight hours. The process is sometimes seen with a second reactor, in which a selective hydrogen combustion takes place along with further dehydrogenation. Presumably a noble metal catalyst is also being used here.

Like in the Oleflex process above, the challenge here is the noble metal cost. It would therefore be desirable to replace the noble metal with a base metal, i.e. a common and inexpensive metal.

It has now been found that dehydrogenation of alkanes is possible using a specific member of a new generation of metal sulfide catalysts, which are easy to manufacture and remain in their active phase during operation. The specific metal sulfide in question is lead(II)sulfide, PbS.

Thus, the present invention relates to a process for the dehydrogenation of alkanes to the corresponding unsaturated alkenes and hydrogen (H₂), wherein the alkane is contacted with a catalyst comprising lead(II)sulfide (PbS) supported on a carrier, said catalyst being regenerated when needed.

The steps for regeneration comprise (a) oxidation in dilute air, (b) conversion into the corresponding sulfate and (c) conversion back to the sulfide by reduction in dilute hydrogen containing some hydrogen sulfide. The oxidation in step (a) is preferably carried out at a temperature between 350 and 750°C, most preferably at a temperature between 400 and 600°C.

Further, the invention relates to a process for the dehydrogenation of alkanes to the corresponding unsaturated alkenes and hydrogen (H₂) comprising contacting the alkane with a catalyst supported on a carrier, said catalyst comprising lead(II)sulfide (PbS). Preferably, the dehydrogenation is carried out at a temperature between 500 and 650°C, most preferably at a temperature between 520 and 620°C.

It is preferred to carry out the dehydrogenation at a pressure from 0.5 bar below ambient pressure to 5 bar above ambient pressure, most preferably at ambient pressure or at a pressure from 0.5 bar below ambient pressure up to ambient pressure.

In the process, the feed gas contains sulfur in an amount which preferably is determined such that the equilibrium reaction PbS + H₂ <-> Pb + H₂S is shifted towards PbS throughout the reactor. It means that enough H₂S must constantly be present in the reactor to secure presence of PbS. Thermodynamic data for the reaction may be found in HSC Chemistry for Windows, Outo-kompi, Finland (2002).

Lead sulfide refers to two compounds containing lead and sulfur, i.e. lead(II) sulfide, PbS, containing lead in the +2 oxidation state, naturally occurring as a mineral named galena, and lead(IV) sulfide, PbS₂, containing lead in the +4 oxidation state. The present invention specifically deals with dehydrogenation catalysts comprising PbS.

The use of lead sulfides as catalysts has so far been quite sparse. However, nickel, cadmium and lead sulfides have been used as catalysts in the vapor phase reduction of nitrobenzene as early as in 1930 (O.W. Brown and R.J. Hartmen, J. Phys. Chem. 34, 2651-2665 (1930)), and especially lead sulfide was found to be an effective catalyst for that purpose.

EP 0 568 303 A2 discloses a catalyst for dehydrogenation of organic compounds, such as alkanes to alkenes, which comprises a sulfided combination of nickel and lead on a base-treated non-acidic support. Sulfur is added to the feed stream in order to maintain catalyst selectivity, but it is not disclosed that this addition shifts the equilibrium towards the metallic sulfide.

US 2.768.931 describes the use of a lead sulfide catalyst in the oil sweetening process known as the Bender™ process, where the oil to be sweetened after being mixed with suitable reagents is passed through a catalyst bed comprising lead(II) sulfide supported on an inert carrier. The lead sulfide catalyst used can subsequently be regenerated as outlined in US 3.117.937. Lead(II) sulfide catalysts for use in oil sweetening processes are further disclosed in US 3.247.089 and US 3.720.627. None of these US patents describe the catalysts as being usable as dehydrogenation catalysts.

Apart from these ancient prior art documents, there seems to be no mention in the literature regarding use of lead sulfide as catalysts. This is probably due to the fact that a number of environmental reasons for not using lead catalysts can be given.

US 2.315.107 discloses the dehydrogenation of alkanes using metal sulfides. There is, however, no disclosure of the use of lead sulfide as catalyst in the dehydrogenation process.

The use of metal sulfides in a broader sense, i.e. where the metal is primarily selected among Zn, Cu, Mn, Mo, Fe, Co and Ni, for isobutane dehydrogenation to isobutene, is known from a number of prior art documents. For instance, processes for preparing alkenes from alkanes using metal sulfide catalysts are described in GB 488.651 (1938) and in US 3.280.210 (1966). More recently, an article by Guowei Wang, Chunyi Li and Honghong Shan (ACS Catal. 4(4), 1139-1143, 2014) and a number of pending Chinese patent applications (CN 104607168 A, CN 104069778 A, CN 104607168 A and CN 103861619 A) deal with the dehydrogenation of alkanes to alkenes and catalysts for that purpose.

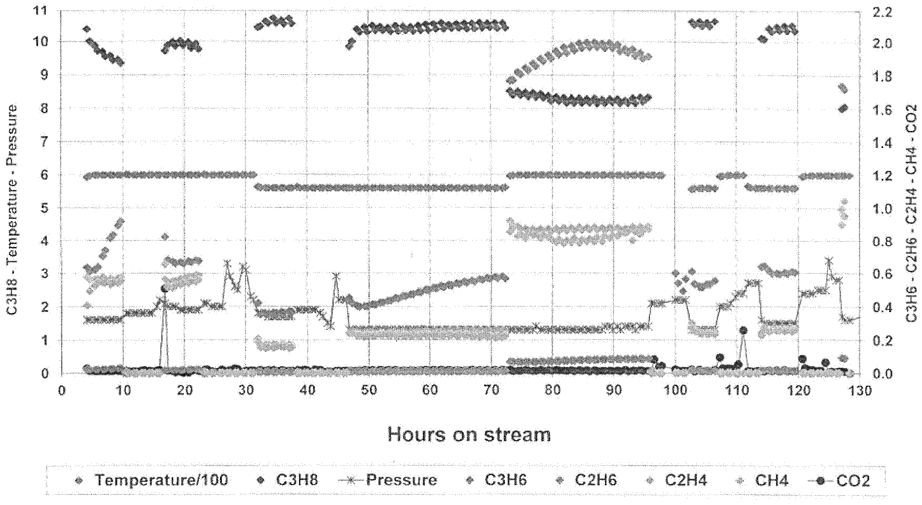

The appended figure shows the dehydrogenation of propane with the use of a catalyst containing 14 wt% Pb (atomic weight 207.2) as PbS. The spent catalyst showed some very large PbS crystals (∼700 Å). Thus, on surface basis, PbS may indeed be very active.

The run illustrated in the figure showed an activity of 130 N1 propene/kg catalyst/h and an activation energy of 1.6 eV.

Repeated tests using lead catalysts have further shown features like a very slow reactivation after regeneration, as seen in the figure. PbSO₄ is one of the most stable sulfates in existence, and a slow reduction of sulfate into sulfide is probably experienced. Other very stable sulfates are ZnSO₄ and SnSO₄.

In dehydrogenation processes, such as the Oleflex process, it is normal practice to add substantial amounts of sulfur to the process in order to protect the material. Thus, in a substantially standard plant, an amount of dimethyl disulfide corresponding to a concentration of H₂S in the gas phase of 20-100 ppm will be used.

The properties of the lead sulfide catalyst used according to the present invention can be summarized as follows: It has a melting point of 1118°C and a Kp(H₂/H₂S) value at 600°C of 247.

The invention is illustrated further in the examples which follow.

The catalyst testings have been carried out in a tubular reactor specifically built for high temperature application, such as tar reforming, and as such it is suitable for testings requiring gases containing sulfides. The reactor has a length of around 100 cm and an internal diameter of 10 mm. The catalysts to be tested are placed on a grid connected to a moveable thermocouple which measures the inlet temperatures. Isothermal control is ensured by four independent heating zones. The tests are carried out using a 10% propane in nitrogen mixture, to which hydrogen, nitrogen and H₂S can be added. The typical H₂S concentration can vary from 50 ppm up to 0.5%, although the low values are associated with some uncertainty due to wall effects. This means that the H₂S/H₂ ratio can be varied from 10⁻³ to 10⁻¹.

The typical test conditions have been a temperature between 560 and 600°C using 5 g of the catalyst to be tested in 2-5 mm fractions. Some of the test gases are listed in the table below. Ideally, the pressure should be low, but due to pressure drop incidents in the system the typical pressure has been between 0.2 and 0.3 MPa. The test cycle will typically conclude with Gas 1 at 600°C, and the evaluation of the activities will be based on their performance at this condition.

**Test gas compositions***

| Gas no | Flow | C₃H₈ % | H₂ % | H₂S % | N₂ % | H₂/H₂S i | H₂/H₂S O |
|---|---|---|---|---|---|---|---|
| 1 | 55 | 9.1 | 8.7 | 0.36 | bal. | 24 | 29 |
| 2 | 51 | 9.8 | 1.8 | 0.20 | bal. | 9 | 19 |
| 3 | 25.5 | 9.8 | 1.8 | 0.20 | bal. | 9 | 19 |
| 4 | 25.5 | 9.8 | - | 0.004 | bal. | 0 | 500 |
| 5 | 55 | 9.1 | 8.2 | 0.9 | bal. | 9 | 11 |
| 6 | 55 | 9.1 | 5.5 | 0.008 | bal. | 0 | 500 |
| 7 | 51 | 9.8 | - | 0.004 | bal. | 0 | 1000 |

| | | | | | | | |
|---|---|---|---|---|---|---|---|
| *) Flow is Nl/h; bal. is balance; I = inlet; o = outlet | | | | | | | |

The effluent gases are measured by gas chromatography.

Blind tests conducted both with an empty reactor and with 5 g of catalyst support gave the same surprising result that the CH₄, C₂H₄ formation was almost solely due to the empty reactor and also that some dehydrogenation took place, most likely at the reactor wall. For the typical test conditions 560-600°C, the amount of propene corresponded, on carbon basis, to the amount of C₁ and C₂. Calculations of the dissociation due to the empty space in the reactor are in good agreement with the experimental results. It is noteworthy that these homogeneous reactions taking place at elevated temperatures most likely are the cause for the reduced selectivity of the industrial reactions, in particular the Oleflex process, in which the gas is pre-heated to 650°C four times before entering the dehydrogenation reactor. The catalytic reaction is highly selective; in fact, when the results are corrected for the empty reactor contribution, a selectivity close to 100% is achieved. Ethane formation was pronounced in the presence of catalysts. However, compared to the amount that should have been present if the equilibrium amount was formed, the hydrogenation of ethylene was far from complete, indicating that ethylene is a primary product from the homogeneous propane dissociation.

The first tests were done using a reactor with a thermowell made in the same material. They showed a severe sulfide corrosion. Thus, a change was made to another reactor and thermowell. This reactor had an internal diameter of 15 mm versus the 10 mm diameter in the former. This made the amount of C₁ and C₂ increase by 50%.

The result reported has been corrected by assuming a selectivity of 50% in the empty reactor. Thus, on carbon basis the carbon in C₁ and C₂ equals the propylene formed by the empty reactor. The rate for the catalyst has been corrected for this contribution.

### Example 1

15 g Pb(NO₃)₂ is dissolved in 37.5 g water. This solution is used to impregnate 50 g of a support (pv = 1 ml/g). The sample is rolled for 1 hour, dried overnight at 100°C and calcined at 500°C for 2 hours (4 hours heating ramp).

The sample is then washed in 100 ml of a 2% K₂CO₃ solution for 1 hour (rolling board). Afterwards the sample is washed two times with 200 ml water (one hour each, rolling board).

The sample is filtered and dried overnight at 100°C. The catalyst contains 14 wt% Pb and 0.8 wt% K.

### Example 2

20 g Pb(CH₃COO)₂·3H₂O is dissolved in 37.5 g water. This solution is used to impregnate 50 g support (pv = 1 ml/g). The sample is rolled for 1 hour, dried overnight at 100°C and calcined at 500°C for 2 hours (4 hours heating ramp).

The sample is then washed in 100 ml of a 2% K₂CO₃ solution for 1 hour (rolling board). Afterwards the sample is washed two times with 200 ml water (one hour each, rolling board). The sample is filtered and dried overnight at 100°C. The catalyst contains 18 wt% Pb and 0.8 wt% K.

### Example 3

15 g Pb(NO₃)₂ and 1.5 g KNO₃ are dissolved in 37.5 g water. This solution is used to impregnate 50 g of a support (pv = 1 ml/g). The sample is rolled for 1 hour, dried overnight at 100°C and calcined at 500°C for 2 hours (4 hours heating ramp). The catalyst contains 14 wt% Pb and 1 wt% K.

### Example 4

20 g Pb(CH₃COO)₂·3H₂O and 1.6 g KNO₃ are dissolved in 37.5 g water. This solution is used to impregnate 50 g of a support (pv = 1 ml/g). The sample is rolled for 1 hour, dried overnight at 100°C and calcined at 500°C for 2 hours (4 hours heating ramp). The catalyst contains 18 wt% Pb and 1 wt% K.

### Example 5

5.0 g of the catalyst prepared in Example 1 was placed in a plug flow tubular stainless steel reactor (1.0 m long and with an internal diameter of 15 mm). The catalyst was placed in the middle of the reactor and supported on a grid. In the top as well as in the bottom of the catalyst bed a thermocouple was placed.

The inlet and exit pressures were recorded by pressure transducers. Before the catalytic tests, blind tests were carried out, and the results from the blind tests were subtracted from the later catalytic tests. The blind tests typically showed 4% conversion at 560°C and 12% conversion at 600°C, both with a selectivity to propene of 50%.

The catalyst was initially reduced and sulfide in a gas consisting of 50 Nl/h of N₂, 4.5 Nl/h of H₂ and 0.5 Nl/h of H₂S, being heated in this gas from room temperature to the reaction temperature of 600°C over a period of 60 minutes.

At 600°C, the catalyst was tested in a gas containing 45 Nl/h of N₂, 5 Nl/h of C₃H₈, 1.8 Nl/h of H₂ and 0.2 Nl/h of H₂S. It showed a formation (after subtraction of the reactor contribution) of 0.2 Nl/h of propene corresponding to 40 Nl/h propene/kg cat/h. The subtraction of the reactor contribution showed a selectivity of 100% within the experimental error. The experiment was conducted at a pressure of 0.26 MPa, and the measurements were recorded after 10 hours of reaction.

### Example 6

After 40 hours of reaction, the catalyst was subjected to regeneration for 6 hours at 560°C in the presence of a gas containing 49.5 Nl/h of N₂ and 0.5 Nl/h of O₂. Then it was reduced and sulfided for 2 hours in a gas containing 50 Nl/h of N₂, 9 Nl/h of H₂ and 1 Nl/h of H₂S. It was then tested for 20 hours at 560°C in a gas containing 45 Nl/h of N₂, 5 Nl/h of propane, 0.9 Nl/h of H₂ and 0.1 Nl/h of H₂S. A formation (after subtraction of the reactor contribution) of 0.21 Nl/h of propene was found at a selectivity of approximately 100% corresponding to 124 Nl/h propene/kg cat/h. The pressure was 0.22 MPa.

The temperature was increased to 600°C using the same reaction mixture. After a reaction time of 85 hours in total, a formation of propene (after subtraction of the reactor contribution) of 0.62 Nl/h was found, corresponding to 124 Nl/h propene/kg cat/h. The pressure was 0.22 MPa.

The catalyst was regenerated after more than 50 consecutive hours in the propane-containing gas. The amount of CO₂ was recorded, and it was concluded that less than 1% of the converted propane had ended up as carbon on the catalyst and the reactor wall.

The catalyst was reduced and sulfide and then tested again at 600°C using the same conditions as above. It still displayed the same performance.

After cooling in nitrogen, the catalyst was analyzed by means of X-ray powder diffraction. Apart from the carrier material, only PbS was seen. It had an average crystallite size of 64 nm.

The catalyst of the invention is deactivated slowly by carbon deposition and therefore it needs to be regenerated, just like the commercially available catalysts based on platinum or chromium oxide. The regeneration takes place by combustion in dilute air, i.e. 1% O₂ and 99% N₂, at 560-600°C.

Regeneration of most metal sulfides using N₂ with 1% O₂ will lead to formation of the corresponding sulfate. In order to conserve sulfur on the catalyst, regeneration should start at 400°C followed by a carbon removal at 600°C.

The regeneration takes the catalyst through two phase transition stages, from sulfide to sulfate or oxide and back again to sulfide. The phase transitions involve not only structural transformations, but also volume changes. It is expected that sintering/dispersion of the system will reach steady state after a number of regenerations.

During dehydrogenation, some carbon is deposited on the catalyst, resulting in a slow deactivation. Dehydrogenation takes place for some hours followed by catalyst regeneration in N₂ containing 1% O₂. This is typically followed by a sulfidation or a direct return to dehydrogenation. In this case, a direct reaction between sulfate and propane takes place, resulting in a large CO₂ formation.

The carrier is treated with a dilute alkali compound and subsequently washed to remove acid sites. Preferably, the dilute alkali compound is potassium carbonate or any other potassium compound.

In the experiments, the carrier has been dipped in a dilute potassium carbonate solution followed by a two-step wash in demineralized water, resulting in a potassium content of 0.15 wt%. Acid sites have been removed, but not necessarily all of them. The results indicate a pressure influence on the carbon formation, and they also indicate that carbon formation takes place from propylene, not propane. Furthermore, the results indicate that there is a complete carbon removal during regeneration.

## Claims

1. A process for the dehydrogenation of alkanes to the corresponding unsaturated alkenes and hydrogen (H₂), wherein the alkane is contacted with a catalyst comprising lead(II) sulfide (PbS) supported on a carrier, said catalyst being regenerated when needed.

2. Process according to claim 1, wherein the dehydrogenation is carried out at a temperature between 500 and 650°C, preferably at a temperature between 520 and 620°C.

3. Process according to claim 1 or 2, wherein the dehydrogenation is carried out at a pressure from 0.5 bar below ambient pressure to 5 bar above ambient pressure.

4. Process according to claim 3, wherein the dehydrogenation is carried out at ambient pressure or at a pressure from 0.5 bar below ambient pressure up to ambient pressure.

5. Process according to any of the claims 1-4, wherein the feed gas contains sulfur in an amount determined such that the equilibrium reaction PbS + H₂ <-> Pb + H₂S is shifted towards PbS throughout the reactor.

6. Process according to claim 1, wherein the steps for regeneration of the catalyst comprise (a) oxidation in dilute air at a temperature between 350 and 750°C, (b) conversion into the corresponding sulfate, and (c) conversion back to the sulfide by reduction in dilute hydrogen containing hydrogen sulfide.

## Patentansprüche

1. Verfahren zur Dehydrierung von Alkanen zu den entsprechenden ungesättigten Alkenen und Wasserstoff (H₂), wobei das Alkan mit einem Katalysator in Kontakt gebracht wird, der auf einem Träger geträgertes Blei-(II)-Sulfid (PbS) umfasst, wobei der Katalysator nach Bedarf regeneriert wird.

2. Verfahren gemäß Anspruch 1, wobei die Dehydrierung bei einer Temperatur zwischen 500 und 650°C, bevorzugt bei einer Temperatur zwischen 520 und 620°C, durchgeführt wird.

3. Verfahren gemäß Anspruch 1 oder 2, wobei die Dehydrierung bei einem Druck von 0,5 bar unterhalb des Umgebungsdrucks bis zu 5 bar über Umgebungsdruck durchgeführt wird.

4. Verfahren gemäß Anspruch 3, wobei die Dehydrierung bei Umgebungsdruck oder bei einem Druck von 0,5 bar unterhalb des Umgebungsdrucks bis Umgebungsdruck durchgeführt wird.

5. Verfahren gemäß einem der Ansprüche 1 - 4, wobei das Einsatzgas Schwefel in einer Menge enthält, die so bestimmt ist, dass die Gleichgewichtsreaktion PbS + H₂ ↔ Pb + H₂S im gesamten Reaktor in Richtung PbS verschoben ist.

6. Verfahren gemäß Anspruch 1, wobei die Schritte zur Regeneration des Katalysators (a) die Oxidation in verdünnter Luft bei einer Temperatur im Bereich von 350 bis 750°C, (b) die Umwandlung in das entsprechende Sulfat und (c) die Rückumwandlung in Sulfid durch Reduktion in verdünntem Wasserstoff, der Schwefelwasserstoff enthält, umfassen.

## Revendications

1. Procédé de déshydrogénation d'alcanes en alcènes insaturés correspondants et hydrogène (H₂), dans lequel l'alcane est mis en contact avec un catalyseur comprenant du sulfure de plomb (II) (PbS) supporté sur un support, ledit catalyseur étant régénéré au besoin.

2. Procédé selon la revendication 1, dans lequel la déshydrogénation est effectuée à une température comprise entre 500 et 650 °C, de préférence à une température comprise entre 520 et 620 °C.

3. Procédé selon la revendication 1 ou 2, dans lequel la déshydrogénation est effectuée à une pression de 0,5 bar en dessous de la pression ambiante jusqu'à 5 bar au-dessus de la pression ambiante.

4. Procédé selon la revendication 3, dans lequel la déshydrogénation est effectuée à pression ambiante ou à une pression de 0,5 bar en dessous de la pression ambiante jusqu'à la pression ambiante.

5. Procédé selon l'une quelconque des revendications 1 à 4, dans lequel le gaz d'alimentation contient du soufre en une quantité déterminée de telle sorte que l'équilibre de la réaction PbS + H₂ ↔ Pb + H₂S est déplacé vers le PbS dans tout le réacteur.

6. Procédé selon la revendication 1, dans lequel les étapes de régénération du catalyseur comprennent (a) l'oxydation dans de l'air dilué à une température comprise entre 350 et 750 °C, (b) la conversion en sulfate correspondant, et (c) la rétro conversion en sulfure par réduction dans de l'hydrogène dilué contenant du sulfure d'hydrogène.
